# EUROPEAN PATENT APPLICATION

(11) **EP 4 656 714 A1**
(43) Date of publication of application: **03.12.2025**
(21) Application number: 25179363.4
(22) Date of filing: 28.05.2025
(51) Int. Cl.: C12N 5/071

(54) **MEDIUM FOR PRODUCING A BILE CANALICULUS AND METHOD FOR PRODUCING A BILE CANALICULUS USING THE SAME**

(30) Priority: 31.05.2024 JP 2024088978
(71) Applicant: INSTITUTE OF SCIENCE TOKYO, Tokyo 152-8550 (JP); Kanto Kagaku Kabushiki Kaisha, Tokyo 103-0023 (JP)
(72) Inventor: Kume, Shoen, Tokyo 152-8550 (JP); Shiraki, Nobuaki, Tokyo 152-8550 (JP); Watanabe, Teruhiko, Kanagawa 2591146 (JP); Fukuda, Tsubasa, Kanagawa 2591146 (JP)
(74) Representative: Meissner Bolte Partnerschaft mbB

(57) **Abstract**

[Object] An object of the present invention is to provide a medium for conveniently forming a bile canaliculus without layering an extracellular matrix such as Matrigel^{®}.

[Solution] The present invention relates to a medium for culturing hepatocytes to form a bile canaliculus comprising a compound that activates K-Ras protein as an active ingredient, and a method for producing a bile canaliculus comprising culturing hepatocytes in the medium.

## Description

### [Technical Field]

The present invention relates to a medium for producing a bile canaliculus and a method for producing a bile canaliculus using the same.

### [Background Arts]

Bile canaliculi, which function as a route of excretion of bile produced in the liver, are also one of the principal routes of elimination of pharmaceuticals. Moreover, drug-induced liver injury caused by bile retention in hepatocytes (cholestasis) due to the inhibition of bile excretion by pharmaceuticals has been one of the major causes of discontinuation of pharmaceutical development. Therefore, in pharmaceutical development, candidate compounds are assessed for bile excretion and for the risk of cholestasis.

In the above-mentioned assessment, in vitro tests using cells (human primary-cultured hepatocytes, iPS-derived hepatocytes, etc.) are performed, though it is generally known that bile canaliculi would not be formed in normal culture (Non-Patent Document 1). Therefore, sandwich (SW)-culture technique is used in conventional bile canaliculus formation methods method in which hepatocytes are sandwiched between extracellular substrates, and among those, Matrigel^{®} SW culture is mainly used in which Matrigel^{®} is layered on top of hepatocytes on collagen I (Non-Patent Documents 1 and 2, Patent Documents 1-3).

The present inventors have developed a method for improving the initial adhesion of hepatocytes by culturing hepatocytes using a medium containing cAMP, a cAMP analog or a substance that increases cAMP concentration to activate cAMP signaling (Patent Document 4). Furthermore, it was confirmed that bile canaliculi can be formed by SW culture of hepatocytes using the developed medium (Non-Patent Document 3).

Besides, with respect to the adhesiveness of hepatocytes, it has been reported that an adherens junction is formed by activating K-Ras protein by oncostatin M (OsM) (Non-Patent Document 4).

In SW culture, in which an extracellular matrix (typically Matrigel^{®}) is layered, there is concern that it is difficult for the test drug to reach hepatocytes. Moreover, since Matrigel^{®} is derived from mouse sarcoma, its composition is indistinct and there is a large difference between lots. Infection by pathogenic substances and immunogenicity are also regarded as problems. In addition, handling of Matrigel^{®} is complicated as it becomes gel at room temperature. Therefore, there is a need for a method for forming bile canaliculi without layering an extracellular matrix (i.e., without using Matrigel^{®}).

Techniques for forming bile canaliculi without using SW culture include, for example, a report that bile canaliculi can be formed using a hepatocyte culture apparatus that promotes accumulation and excretion of liver metabolites in bile canaliculus-like structures by co-culturing a culture containing a plurality of hepatocytes (first cell culture) with another cell culture that is capable of increasing the excretion activity for liver metabolites in the first culture (second cell culture) (Patent Document 5); and a report that it is possible to form bile canaliculi by co-culturing HeLa cells (HeLa/CLDNs) with HepG2 cells (a human liver cancer cell line, HepG2/CLDNs), both have been forced to express claudin proteins (CLDNs) that are involved in the formation of bile canaliculi (Non-Patent Document 5), etc.

However, these techniques that do not use SW culture are not necessarily sufficient from the viewpoint of practicality because they require special culture equipment and co-culturing.

### [Prior Art Documents]

### [Patent Documents]

[Patent Document 1] JP A 2013-117411
[Patent Document 2] JP A 2003-502016
[Patent Document 3] JP A 2008-503204
[Patent Document 4]WO 2022/138922
[Patent Document 5] WO 2016/158417

### [Non-Patent Documents]

[Non-Patent Document 1] B. Swift et al., "Sandwich-Cultured Hepatocytes: An In Vitro Model to Evaluate Hepatobiliary Transporter-Based Drug Interactions and Hepatotoxicity", Drug Metab Rev. 2010, 42(3), p446-471.
[Non-Patent Document 2] LeCluyse et al., "Formation of extensive canalicular networks by rat hepatocytes cultured in collagen-sandwich configuration", Am J Physiol Cell Physiol, 1994, vol.266, p1764-1774.
[Non-Patent Document 3] Helena, G.A. et al., "Activation of cAMP (EPAC2) signaling pathway promotes hepatocyte attachment", Sci Rep 13, 12352 (2023).
[Non-Patent Document 4] Matsui, T et al., "K-Ras mediates cytokine-induced formation of E-cadherin-based adherens junctions during liver development", EMBO J., 2002, 21, p.1021-1030
[Non-Patent Document 5] Hiroshi Arakawa et al., "Induction of open-form bile canaliculus formation by hepatocytes for evaluation of biliary drug excretion", Commun. Biol., 2023, 22; 6(1)

### [Summary of the Invention]

### [Problems to be Solved by the Invention]

An object of the present invention is to provide a medium for conveniently forming bile canaliculi without layering an extracellular matrix such as Matrigel^{®}.

### [Means to Solve the Problem]

In the course of intensive research to solve the above-mentioned problems, the present inventors have found that the formation of bile canaliculi can be promoted without layering an extracellular matrix such as Matrigel^{®} by culturing hepatocytes using a medium containing as an active ingredient a compound that activates K-Ras protein. The present inventors further continued the research and completed the invention.

The present invention relates to the following inventions:
[1] A medium for culturing hepatocytes to form a bile canaliculus, comprising as an active ingredient a compound that activates K-Ras protein.
[2] The medium according to [1], wherein the compound that activates K-Ras protein is oncostatin M (OsM), an interleukin (IL)-6 family cytokine or a guanine nucleotide exchange factor (GEF) for K-Ras.
[3] The medium according to [1] or [2], wherein the medium comprises no albumin or less than 0.1 w/v% albumin.
[4] The medium according to any one of [1] to [3], wherein the medium does not comprise one or more selected from the group consisting of bovine serum albumin (BSA), 6-bromoindirubin-3'-oxime (BIO) and calcitriol.
[5] The medium according to any one of [1] to [4], wherein the medium comprises none of cAMP, cAMP analogs, and substances that increase intracellular cAMP concentration.
[6] A method for producing a bile canaliculus, comprising a step of culturing hepatocytes in a medium according to any one of [1] to [5].
[7] The method for producing a bile canaliculus according to [6], wherein the hepatocytes are primary-cultured hepatocytes.
[8] The method for producing a bile canaliculus according to [6] or [7], comprising a step of replacing the medium 1 to 8 hours after starting the culturing.
[9] The method for producing a bile canaliculus according to any one of [6] to [8], wherein the method does not comprise a step of layering a solubilized basal membrane extracted from Engelbreth-Holm-Swarm (EHS) mouse sarcoma onto the hepatocytes.
[10] The production method according to any one of [6] to [8], wherein the method does not comprise a step of layering an extracellular matrix onto the hepatocytes.

### [Advantageous Effect of the invention]

The bile canaliculi from hepatocytes cultured using the medium of the present invention have a function superior to that of bile canaliculi formed by Matrigel^{®} sandwich (SW) culture method using commercially available media that has been commonly used so far (conventional method). In particular, many of the bile canaliculi formed by using the medium of the present invention often have more elongated form as compared to those formed by the conventional method.

According to the method that use the medium of the present invention, bile canaliculi that are essential for bile excretion/stasis tests can be formed without layering an extracellular matrix such as Matrigel^{®}, so that there is no concern that a drug used in each test is trapped in the extracellular matrix before it reaches the hepatocyte. Therefore, by using the bile canaliculus formed by the method of the present invention, more reliable data can be obtained as compared to the test data in a cellular model constructed using prior art. Moreover, since no extracellular matrix such as Matrigel^{®} is used, the composition of the medium is clear, which can facilitate an analysis of its mechanism and can contribute to further research progress in this field. Furthermore, in addition to making the culture very simple, there is another advantage in terms of cost reduction by not using Matrigel^{®}.

### [Brief Description of the Drawings]

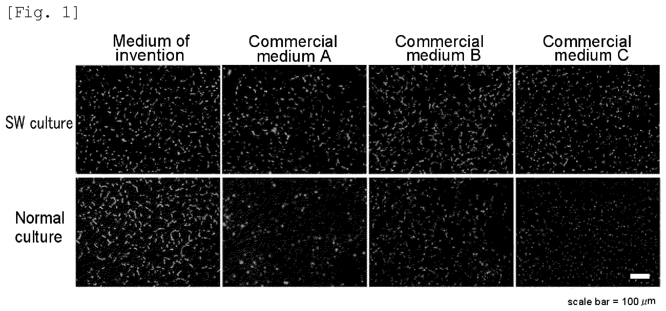

[FIG. 1] FIG. 1 is a diagram showing MRP2 immunostaining images of hepatocytes cultured (either in SW culture or in normal culture) until Day 7 in one of the medium of the present invention and commercially available media A to C.

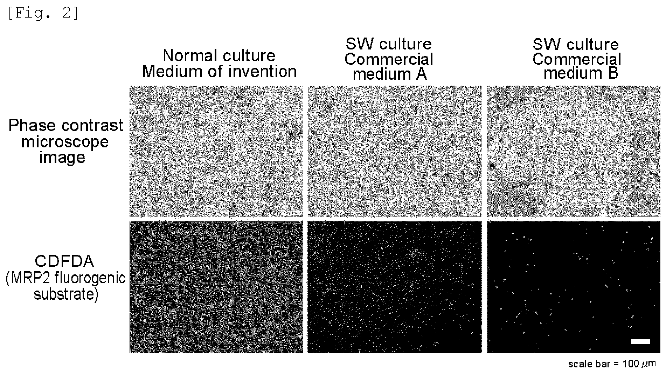

[FIG. 2] FIG. 2 is a diagram showing a phase contrast microscope image and an accumulation of CDFDA in bile canaliculi (fluorescent microscope image) on Day 7 of culture in the medium of the present invention (normal culture) or in commercially available medium A or B (SW culture).

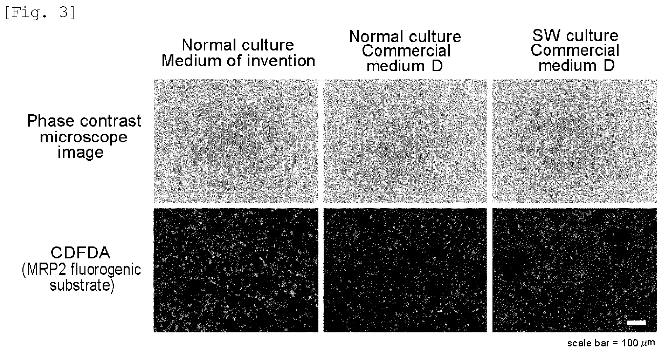

[FIG. 3] FIG. 3 is a diagram showing a phase contrast microscope image and an accumulation of CDFDA in bile canaliculi (fluorescent microscope image) on the Day 4 of culture, either in the medium of the present invention (normal culture), or in the commercial medium D (normal culture or SW culture).

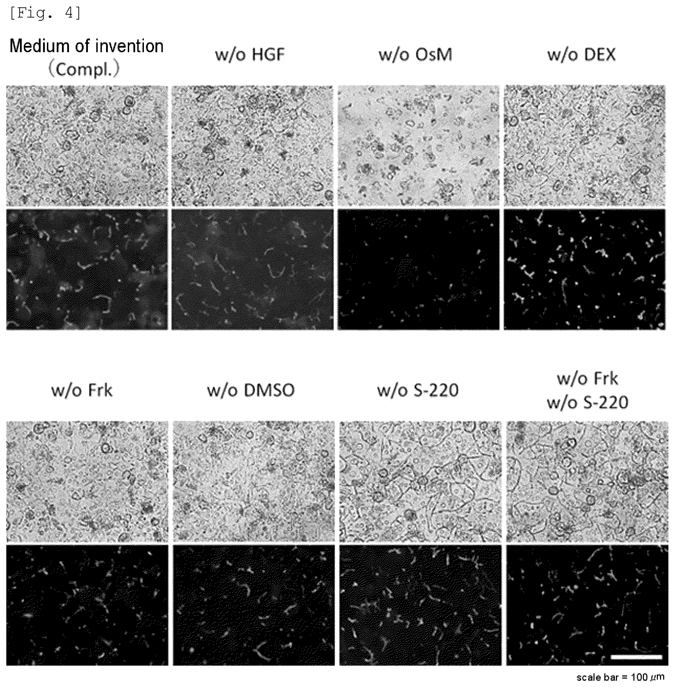

[FIG. 4] FIG. 4 is a diagram showing a phase contrast microscope image (upper row) and an accumulation of CDFDA in bile canaliculi (fluorescent microscope image) on the Day 4 of culture, either in the medium of the present invention or in the same medium from which one or two components had been removed.

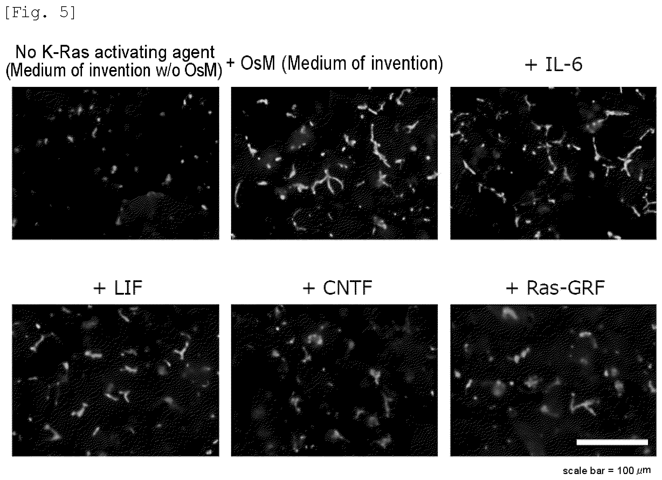

[FIG. 5] FIG. 5 is a diagram showing an accumulation of CDFDA in bile canaliculi (fluorescent microscope image) from hepatocytes on the Day 4 of culture (normal culture) , either in the medium of the present invention without OsM (a negative control), in the medium of the present invention (a positive control), or in the medium of the negative control supplemented with IL-6, LIF, CNTF or RAS-GRF.

### [Description of Embodiments]

The medium of the present invention is a medium for culturing hepatocytes to form a bile canaliculus, and comprises as an active ingredient a compound that activates K-Ras protein.

The hepatocyte is preferably a human-derived hepatocyte, though it may be a hepatocyte derived from a non-human animal. Examples of non-human animals include, for example, mice, rats, guinea pigs, hamsters, rabbits, cats, dogs, sheep, cows, horses, goats, monkeys, and the like.

Hepatocytes differentiated and induced from pluripotent stem cells or hepatocytes further matured from such hepatocyte may be used, though preferably hepatocytes collected from living organisms such as primary-cultured hepatocytes are used. Here, the meaning of "primary-cultured hepatocytes" can be interpreted as the meaning which is usually used by those skilled in the art, though "primary-cultured hepatocytes" can also be defined as hepatocytes that are prepared by inoculating and culturing tissues or cells for the first time after collecting them from living organisms.

There are two types of primary-cultured hepatocytes, a plateable type and a suspension type, and either type of primary-cultured hepatocytes may be used in the present invention.

The compound that activates K-Ras protein is not particularly limited, though it includes, for example, oncostatin M (OsM), interleukin (IL)-6 family cytokines, guanine nucleotide exchange factor (GEF) for K-Ras, and the like. IL-6 family cytokines include, for example, IL-6, IL-11, IL-27, IL-35, IL-39, leukemia inhibitory factor (LIF), ciliary neurotrophic factor (CNTF), cardiotrophin 1 (CT-1), cardiotrophin-like cytokine factor 1 (CLCF1), and the like. GEFs for K-Ras proteins include, for example, CDC25, SDC25, Ras-GRF, SOS, and the like. In particular, OsM or IL-6 family cytokines are preferred.

The medium of the present invention comprises a compound that activates K-Ras protein in a general basal medium.

The basal medium can be, for example, BME medium, BGjB medium, CMRL 1066 medium, Glasgow MEM medium, Improved MEM medium, IMDM medium, Medium 199 medium, Eagles MEM medium, αMEM medium, DMEM medium, Ham's medium, RPMI 1640 medium, Fischer's medium, William's E medium, or a mixed medium thereof, though it is not particularly limited as long as it is a medium that can be used for culturing animal cells. These media are commercially on the market and available.

The medium of the present invention may contain other additives, such as, for example, lipids, amino acids (e.g., non-essential amino acids), vitamins, sugars, hormones (e.g., dexamethasone), growth factors (e.g., HGF), cytokines, insulin, transferrin, antioxidants, serum substitutes, 2-mercaptoethanol, pyruvic acid, dimethyl sulfoxide (DMSO), adenylylcyclase activator (e.g., forskolin), Epac activator (e.g., Sp-8-BnT-cAMPS (S-220)), buffering agents (e.g., HEPES), inorganic salts, antibiotics (e.g., penicillin or streptomycin) or antibacterial agents (e.g., amphotericin B).

In one embodiment, the medium of the present invention comprises no albumin or less than 0.1 w/v% albumin. By limiting the amount of albumin in the medium, it is possible to improve the adhesiveness of hepatocytes and to maintain or improve the function of hepatocytes. The albumin concentration in the medium may be less than 0.1 w/v%, though it is preferably 0.03 w/v% or less, more preferably 0.01 w/v% or less. Here, the function of hepatocytes means, for example, the activity of enzymes such as cytochrome P 450 (e.g., CYP3A4).

In one embodiment, the medium of the present invention does not comprise one or more selected from the group consisting of bovine serum albumin (BSA), 6-bromoindirubin-3'-oxime (BIO) and calcitriol. Here, bovine serum albumin (BSA) also includes bovine serum albumin (fatty acid-free) (BSA-FAF). Since these components are not recognized to contribute to bile canaliculus formation, there is no need to include them as essential components in the medium of the present invention.

In one embodiment, the medium of the present invention comprises none of cAMP, cAMP analogs, and substances that increase intracellular cAMP concentration. Since these components are not recognized to contribute to bile canaliculus formation, there is no need to include them as essential components in the medium of the present invention.

Examples of cAMP analogs include, for example, cNMPS-Sp, 6-Bnz-cAMP, 8-Bromo-cAMP, Dibutyryl-cAMP, 8-CPT-2Me-cAMP, 8-pCPT-2-O-Me-cAMP-AM and the like.

Substances that increase intracellular cAMP concentration include phosphodiesterase inhibitors and adenylyl cyclase activators. Phosphodiesterase inhibitors include IBMX (3-isobutyl-1-methylxanthine), theophylline, papaverine, caffeine, rolipram, sildenafil, milrinone, cilostazol, vinpocetine, theobromine, resveratrol and the like. Examples of adenylyl cyclase activators include forskolin.

The method for producing a bile canaliculus of the present invention comprises a step pf culturing hepatocytes in the medium of the present invention as described above.

In one embodiment, in the method for producing a bile canaliculus of the present invention, the hepatocytes are primary-cultured hepatocytes.

In the culture method of the present invention, it is preferable to replace the medium 1 to 8 hours after starting culture. By such medium replacement, it is possible to improve the adhesiveness of hepatocytes and to maintain or improve the function of hepatocytes. The timing of medium replacement may be 1 to 8 hours after starting culture, though it is preferably 3 to 5 hours after the starting culture, and more preferably 3.5 to 4.5 hours after starting culture.

The culture method of the present invention is performed at a culture temperature suitable for culturing hepatocytes (usually 30 to 40 °C, preferably about 37 °C), and is usually in a CO₂ incubator. The specific culture period is usually 0.5 to 30 days, preferably 1 to 14 days. More preferably, it is 3 to 7 days.

In one embodiment, the method for producing a bile canaliculus of the present invention does not comprise a step of layering a solubilized basal membrane extracted from Engelbreth-Holm-Swarm (EHS) mouse sarcoma onto the hepatocytes. Here, examples of the solubilized basal membrane extracted from Engelbreth-Holm-Swarm (EHS) mouse sarcoma include Matrigel^{®} (registered trademark) (Corning) and the like.

In one embodiment, the method for producing a bile canaliculus of the present invention does not comprise a step of layering an extracellular matrix onto hepatocytes. Examples of the extracellular matrix include, apart from the solubilized basal membrane extracted from Engelbreth-Holm-Swarm (EHS) mouse sarcoma, collagen.

Hereinafter, the present invention will be described in detail with reference to examples, though the present invention is not limited to these examples.

### [Examples]

### 1. Materials and Methods

### [Preparation of the medium of the present invention]

The medium of the present invention was prepared according to the medium composition of Table 1 using William's E Cica-modified 2 (BioConcept) as a basic medium.

**[Table 1] Composition of the medium of the present invention**

| Name of Ingredients | Final Concentration |
|---|---|
| William's E cica modified-2 | |
| Sodium bicarbonate | 1.26 g/L |
| HEPES | 12.75 mM |
| Penicillin/Streptomycin | 100 units/mL |
| Insulin (Human, Recombinant) | 5 mg/L |
| Transferrin (Human, Holo) | 5 mg/L |
| Recombinant Human HGF | 10 ng/mL |
| Recombinant Human Oncostatin M | 10 ng/mL |
| Dexamethasone | 10 µmol/L |
| Forskolin | 10 µmol/L |
| Dimethyl Sulfoxide (DMSO) | 0.8% |
| S-220 | 3 µmol/L |

### [Hepatocyte normal culture]

The frozen stock of human primary-cultured hepatocytes (PHHs) was thawed using commercially available thawing medium, and cell number was counted using trypan blue. To a 96-well plate coated with collagen I, PHHs were plated at 1.0 × 10⁵ cells/well seeding density, using the medium of the present invention, commercial medium A, commercial medium B, and commercial medium C such that the medium volume was 75 µL/well, and cultured at 37 °C under 5% CO₂ condition. Four hours, 1 day, 2 days, and 4 days after seeding, the total amount of medium was replaced using each medium such that the medium volume was 75 µL/well, and the plate was cultured until Day 7 of culture.

### [Culturing human liver chimeric mouse-derived hepatocytes]

The process of seeding human liver chimeric mouse-derived hepatocytes (HepaSH^{®}) was in accordance with the recommended protocol by the manufacture. The cells were seeded onto a 96-well plate coated with collagen I at 1.0 × 10⁵ cells/well seeding density in the medium of the present invention or a medium recommended by the manufacture such that the volume of the medium is 75 µL/well, and normally cultured at 37 °C under 5% CO₂ condition. Four hours, 1 day, 2 days, 3 days, and 4 days after seeding, the whole medium was replaced using the medium of the present invention or the commercial medium D such that the volume of the medium is 75 µL/well, and the plate was cultured until Day 4 of culture . In addition, in SW culture condition, the medium was replaced 1 day after seeding using commercial medium D containing Matrigel^{®} at the final concentration of 0.25 mg/mL, and 4 hours, 2 days, 3 days and 4 days after seeding using commercial medium D that did not contain Matrigel^{®}, such that the volume of the medium is 75 µL/well.

### [Preparation of Matrigel^{®} containing medium]

Matrigel^{®} was added at a final concentration of 0.25 mg/mL to each of the medium of the present invention and the commercial media A, B and C.

### [Matrigel^{®} sandwich (SW) culture of hepatocytes]

The frozen stock of PHHs was thawed using commercially available thawing medium, and cell number was counted using trypan blue. To a 96-well plate coated with collagen I, PHHs were seeded at 1.0 × 10⁵ cells/well seeding density, using the medium of the present invention, commercial medium A, commercial medium B, and commercial medium C such that the medium volume was 75 µL/well. Four hours after seeding, the medium was replaced with each medium, and after 1 day, it was replaced with a Matrigel^{®} containing medium under each condition (75 µL/well). Subsequently, 2 days and 4 days after starting culture, the medium was replaced using either the medium of the present invention without Matrigel^{®}, or the commercial medium A, B or C, at a medium volume of 75 µL/well.

### [Confirming bile acid excreting transporters expression by immunostaining]

On Day 7 of culture, the medium was removed, 4% paraformaldehyde (PFA) was added at 100 µL/well, and the culture was allowed to stand at room temperature for 15 minutes, and then washed three times with PBS (-) (cell fixation). 1% Triton-X 100 was added at 100 µL/well, the plate was allowed to stand at room temperature for 10 minutes, and then washed once with PBS (-). Blocking buffer was added at 200 µL/well, the plate was allowed to stand at room temperature for 1 hour, then the blocking buffer was removed, and anti-MRP2 antibody was added at 100 µL/well. On the next day, the primary antibody solution was removed, the plate was washed three times with PBS (-), and then the secondary antibody was added at 100 µL/well, and the plate was allowed to stand at room temperature for 2 hours under a shading condition. After standing still, the plate was washed three times with PBS (-) and observed using fluorescence microscopy.

### [Excretion of fluorescent substrates into bile canaliculi by bile acid excreting transporters]

On day 7 of culture, the medium was removed, 4 µmol/L CDFDA solution was added at 100 µL/well, and the plate was incubated in a CO₂ Incubator (5% CO₂, 37 °C) for 10 minutes. Then, the plate was washed three times with HBSS(+), a medium of each condition was added at 75 µL/well, incubated in the incubator (5% CO₂, 37 °C) for 10 minutes, and observed using fluorescence microscopy.

### [Verifying the effect of each component of the medium of the present invention in the formation of bile canaliculi]

Eight types of media in total: the medium of the present invention, and media prepared by removing the main six components (HGF, OsM, DEX, Frk, DMSO, S-220) from the medium of the present invention (in some case, 2 components were removed), were prepared, and each was used for performing normal culture. Phase contrast microscopy was performed on Day 4 of culture. Moreover, on Day 4 of culture, the medium was removed, a 4 µmol/L CDFDA solution was added at 100 µL/well, and the culture was incubated for 10 minutes in CO₂ incubator (5% CO₂, 37 °C). Thereafter, the culture was washed three times with HBSS(+), the medium for each condition was added at 75 µL/well, then the culture was incubated in CO₂ incubator (5% CO₂, 37 °C) for 10 minutes and observed by fluorescence microscopy.

### [Verifying effectiveness of different K-Ras activating agents in the formation of bile canaliculi]

Two types of media were used as controls: the medium of the present invention without OsM (a negative control), and the medium of the present invention (a positive control), and four more types of media were prepared based on the negative control to which a K-Ras activating agent other than OsM (IL-6, LIF, CNTF or Ras-GRF) was added (six types of media in total). Each medium was used for carrying out normal culture, and on culture Day 4 the medium was removed and 4 µmol/L CDFDA solution was added at 100 µL/well. The culture was incubated in a CO2 incubator (5% CO₂, 37 °C) for 10 minutes, and observed using fluorescence microscopy.

### 2. Results

### [Confirming bile acid excreting transporter expression by immunostaining]

Under the conditions where the cells were normally cultured in the medium of the present invention, remarkable expression of MRP2 was observed. On the other hand, under the conditions where the cells were normally cultured in the commercial medium A, B or C, almost no expression of MRP2 was observed. In addition, it was observed that more MRP2 was expressed in the condition where the cells were normally cultured in the medium of the present invention than that under the conditions where SW culture was performed using the commercial medium A, B or C (conventional method) (FIG. 1).

Even in the case where SW culture was performed using the medium of the present invention, an equal level or higher expression of MRP2 was observed compared to the case of SW culture in the commercial medium A, B or C. When this was compared with the case of normal culture in the medium of the present invention, the expression of MRP2 was higher in the case of normal culture.

### [Excretion of fluorescent substrates into bile canaliculi by bile acid excreting transporters]

On Day 7 of culture, remarkable accumulation of CDFDA in the capillaries was observed under the normal culture condition in the medium of the present invention as compared with the conditions where Matrigel^{®} SW culture was performed using commercial medium A or B (conventional method) (FIG. 2).

### [Verifying effectiveness of the present medium on human liver chimeric mouse-derived hepatocytes]

In the fluorescent microscope images on culture Day 4, a dotted accumulation of CDFDA in bile canaliculi was observed under normal culture condition in the commercial medium D, and a tubular accumulation of CDFDA in bile canaliculi was partly observed under SW culture condition in the commercial medium D. On the other hand, in the medium of the present invention, a tubular accumulation of CDFDA in bile canaliculi was observed throughout the viewing field (FIG. 3).

### [Verifying the effect of each component of the medium of the present invention in the formation of bile canaliculi]

In the phase contrast microscope image on Day 4 of culture, boundaries between cells could hardly be observed under the conditions where the medium did not contain OsM. On the other hand, under other conditions, boundaries between cells were observed. In addition, in the fluorescence microscopy image on Day 4 of culture, almost no accumulation of CDFDA in the bile canaliculi was observed under the condition where the medium did not contain OsM. On the other hand, accumulation of CDFDA in bile canaliculi was observed under other conditions (FIG. 4).

### [Verifying effectiveness of different K-Ras activating agents in the formation of bile canaliculi]

In the fluorescent microscope images on culture Day 4, dotted accumulation of CDFDA in bile canaliculi was observed in the negative control, whereas tubular accumulation of CDFDA in bile canaliculi was observed under the conditions using four types of K-Ras activating agent-containing media. In particular, under the condition where IL-6 was supplemented, a comparable level of accumulation of CDFDA was observed to that of OsM (the positive control) (FIG.5).

### 3. Discussion

From the above results under the condition where the medium does not contain OsM, it was suggested that OsM contributes to the promotion of bile canaliculus formation. Moreover, from the above-described results in the verification of the effectiveness of K-Ras activating agents, it is thought that OsM reinforces the adhesion between cells via activation of K-Ras protein that is involved in the formation of intercellular adhesive binding. It is considered that the formation of bile canaliculi is thus promoted without performing sandwich culture. Furthermore, it is presumed that, by seeding primary-cultured hepatocytes (PHHs) in a medium that does not contain albumin, PHHs quickly adhere to the culture equipment, so that the damage of thawing and seeding is reduced, maintaining a relatively high functional state. It is thought that this point also contributes to the promotion of the formation of bile canaliculi.

### [Industrial Applicability]

The present invention can easily and efficiently form a bile canaliculus from hepatocytes without using a sandwich culture method in which an extracellular matrix is layered. The bile canaliculus produced according to the present invention is relatively homogeneous and can be used in a wide range of fields, including tests and researches such as screening of candidate drugs.

## Claims

1. A medium for culturing hepatocytes to form a bile canaliculus, comprising as an active ingredient a compound that activates K-Ras protein.

2. The medium according to Claim 1, wherein the compound that activates K-Ras protein is oncostatin M (OsM), an interleukin (IL)-6 family cytokine or a guanine nucleotide exchange factor (GEF) for K-Ras.

3. The medium according to Claim 1, wherein the medium comprises no albumin or less than 0.1 w/v% albumin.

4. The medium according to Claim 1, wherein the medium does not comprise one or more selected from the group consisting of bovine serum albumin (BSA), 6-bromoindirubin-3'-oxime (BIO) and calcitriol.

5. The medium according to Claim 1, wherein the medium comprises none of cAMP, cAMP analogs, and substances that increase intracellular cAMP concentration.

6. A method for producing a bile canaliculus, comprising a step of culturing hepatocytes in a medium according to any one of Claims 1 to 5.

7. The method for producing a bile canaliculus according to Claim 6, wherein the hepatocytes are primary-cultured hepatocytes.

8. The method for producing a bile canaliculus according to Claim 6, comprising a step of replacing the medium 1 to 8 hours after starting the culturing.

9. The method for producing a bile canaliculus according to Claim 6, wherein the method does not comprise a step of layering a solubilized basal membrane extracted from Engelbreth-Holm-Swarm (EHS) mouse sarcoma onto the hepatocytes.

10. The production method according to Claim 6, wherein the method does not comprise a step of layering an extracellular matrix onto the hepatocytes.
